# EUROPEAN PATENT SPECIFICATION

(11) **EP 1 255 519 B1**
(45) Date of publication and mention of the grant of the patent: **27.07.2005**
(21) Application number: 01908795.6
(22) Date of filing: 02.02.2001
(51) Int. Cl.: A61F 7/02, A61F 7/10, A61F 5/01, C09K 5/18

(54) **MULTI-PURPOSE DRUG AND HEAT THERAPY TREATMENT SYSTEM**
ALLZWECK -MEDIKAMENT UND -WÄRMEBEHANDLUNGSSYSTEM
MEDICAMENT ET SYSTEME DE THERMOTHERAPIE POLYVALENT

(30) Priority: 08.02.2000 US 181048 P; 08.01.2001 US 756059
(43) Date of publication of application: 13.11.2002
(73) Proprietor: DVORETZKY, Israel, Hamden, CT 06514 (US); Kuleza, John E., Berlin, CT 06037 (US)
(72) Inventor: DVORETZKY, Israel, Hamden, CT 06514 (US); Kuleza, John E., Berlin, CT 06037 (US)
(74) Representative: Brown, Michael Stanley
(86) International application number: PCT/US2001/003432
(87) International publication number: WO 2001/058408

(56) References cited:
- EP-A- 0 970 669
- WO-A-97/12570
- US-A- 5 662 096
- US-A- 5 697 962
- US-A- 5 918 590
- US-A- 5 935 157
- US-A- 5 986 163
- US-A- 6 013 097

## Description

### TECHNICAL FIELD

This invention relates to a treatment system for a wide variety of medical problems providing and, more particularly, to a treatment system combining an exothermic pad with holding means for providing controlled, reproducible, continuous heat delivery to the surface of the skin or subcutaneously at a substantially constant temperature for specific periods of time in order to enable medicinal heat benefits to be realized.

### BACKGROUND ART

It has been found that many medical problems which affect individuals can be successfully treated by applying heat to the affected area. Although a variety of devices have been created for use by individuals to provide heat to a particular area of the body, no system has been achieved which is capable of providing reliable, controlled, repeatable delivery of a precise temperature level for a given length of time in an easily used and convenient form.

The use of heat therapy for many transitory problems, such as pains, muscle pulls, etc., has been practiced for many years. Typically, if heat is employed, these problems are treated with electrically heated pads, hot towels, hot compresses and/or exothermic heating pads. However, in spite of the desirability of using heat in various locations on an individual, no prior art system has been developed which adequately satisfies this need.

One of the prime examples of a medical condition that has recently been found to be treatable with heat therapy is warts. Although the present invention is not limited to warts, the efficacy of using heat for wart treatment is detailed herein as an example of the benefit of the present invention.

Warts are very common viral induced growths (infectious process) and have long presented problems to individuals due to the pain, discomfort and the cosmetic problems associated therewith. Although various methods and treatments have been developed over the years for reducing or eliminating the undesirable effects associated with warts, these prior art applications, treatments and methods have been incapable of eliminating or curing warts or the problems associated with them.

In order to understand the treatment of warts, it is important to know that the skin is divided into three layers, the epidermis, the dermis, and the subcutaneous tissue. The outer layer of the skin is called the epidermis, and varies in thickness from about 0.3 mm on the eyelids and flexural areas to 1.55 mm on the palms and soles. The outermost layer of the epidermis is the stratum corneum (horny layer), which is comprised of completely keratinized dead cells. The thickness of the stratum corneum varies greatly on different parts of the body, being thickest on the palms and soles and totally absent on the oral mucosa.

The bottommost layer of the epidermis, called the basal cell layer, rests upon the basement membrane separating the epidermis from the dermis. The basel cells are the epidermal cells that proliferate. The basal cells proliferate and, by cell division, form the keratinocytes (squamous cell layer, stratum spinosum, spinous layer). The keratinocytes synthesize insoluble protein which remains in the cells and will eventually become a major component of the outer layer (the stratum corneum, horny layer). The keratinocytes continue to divide and to migrate from the bottommost layer to the outermost layer, until the cells finally die. In this process of keratinization, the cells continue to flatten and their cytoplasm appears granular (stratum granulosum, granular layer) until they finally die as they reach the surface to form the stratum corneum (horny layer).

Warts are known to be intra-epidermal tumors of the skin caused by infection with the human papilloma virus (HPV). The HPV induces an abnormal increase of cells in the skin tissue, commonly referred to as hyperplasia, with the hyperplasia being limited to the squamous epithelium. Typically, replication of the papilloma virus is confined to the nuclei of the upper layer of infected epidermal cells.

Many different methods and application systems have been developed to treat warts. However, none of them are uniformly effective. The most common treatments for eliminating warts are surgery, such as conventional surgery, laser surgery, and cryosurgery, the application of different acidic or caustic chemicals in order to completely remove the affected area, and other topical substances, and immune response modifiers, such as Interferon imiquimod, DNCB etc. However, for many individuals, these processes are as difficult or as uncomfortable as the wart condition itself. The warts may return, and patients may be left with pain and/or scar formation. Consequently, many individuals avoid these treatments and, instead, merely accept the discomfort associated with their warts. Unfortunately, when warts are left alone, they may also spread, as a viral-infectious process.

Various chemical compositions have been developed in an attempt to eliminate or reduce the size of the warts by inactivating or slowing the growth of the virus within the skin. In addition, other chemical compounds have been used to reduce or eliminate the overgrowth and keratinization of the wart and thereby lessen its sensitivity or tenderness. Unfortunately, these chemical compositions have been incapable of providing a universally successful treatment for warts and, at best, have only been partially successful.

It has long been known that temperature is an important factor in the development of warts, as well as in treating warts. It is also known that the vast majority of common and plantar warts are produced by HPV types 1, 2, 3, and 4. These viruses prefer to produce lesions on the acral (end) parts of the body which are cooler than the rest of the body. Since the skin surface in general and the extremities of individuals, such as hands and feet, typically have temperatures of between about 24° and 31°C., (less than the body temperature) these locations are typical for the development of the wart virus. In contrast, the anogenital warts and laryngeal warts tend to replicate in more warmer locations and are clearly well known as comprising a different HPV types.

Various studies have shown that the local application of heat causes the virus organisms to be slowed or inactivated. One attempt to effectively apply heat as a wart treatment was the use of hot water baths, with the individual immersing the entire body part containing the wart into a hot water bath. In view of the inherent limitations of this system as well as its limited success, wide acceptance of hot water exposure was never realized.

In particular, one principal limitation found with hot water baths is the inherent difficulty in maintaining a constant temperature for long time periods. In addition, hot water baths are extremely difficult to employ since the entire body part, not only the wart, must be immersed into the hot water. This is particularly difficult since long time exposures are required, as well as repeated exposures several times a day. Furthermore, the hot water bath procedure is particularly hazardous to children due to the risk of severe burning.

Finally, hot water baths are not realistically practical due to the importance of precision in the application temperature. The application temperature is extremely important, since the leeway between a therapeutic temperature and a destructive temperature is very narrow. At 44°C., a six-hour exposure is needed to cause blistering or irreversible damage to the basal layer of the epidermis. However, at 51°C., an exposure of between about 3 to 5 minutes is sufficient to destroy the epidermis.

As is evident from this discussion, warts exemplify the numerous conditions that can be effectively treated if a dependable delivery system for providing heat therapy were available. Similarly, these numerous other medical problems are also effectively treated using a similar dependable delivery system which provides controlled heat therapy.

Therefore, it is a principal object of the present invention to provide a treatment system constructed for cooperative association with a heat delivery patch or exothermic pad and holding member to provide heat therapy which is easy to use and is highly effective in providing controlled temperature levels to any desired location on the skin or body of the user.

Another object of the present invention is to provide a treatment system having the characteristic features described above which is capable of providing a constant level of heat for extended periods of time by controlling air flow and heat insulation.

Another object of the present invention is to provide a treatment system having the characteristic features described above which is easily used by a consumer and enables the treatment system to be retained in any desired position or location for any desired length of time.

In US Patent Specification No. 6,013,097, there is described a treatment device comprising a holding and supporting member constructed for being retained on a desired site of a human body and a heat-generating member positioned in cooperating relationship with the holding and supporting member for enabling the application of heat directly to the desired site. Such a treatment device is hereinafter referred to as a treatment device of the kind specified.

The particular form of treatment device described in US Patent Specification No. 6,013,097 is for the treatment of wounds and includes a foamed ring to ensure that a treatment volume is maintained between the wound surface and the heat-generating member. The foamed ring may be impregnated with an antibiotic, antifungal or antimicrobial material.

It is an object of the present invention to provide an improved treatment device of the kind specified, particularly one that provides enhanced benefits as a result of the inclusion therein of the heat-generating member.

### Summary of the Invention

According to the present invention there is provided a treatment device of the kind specified which is characterised by the the device further comprising a systemic medication that exhibits improved or enhanced penetration from the application of a heat gradient thereto and is selected from the group consisting of corticosteroids, chemotherapeutic agents, antihistamines, anti-parasitics, anti-oxidants, immunomodulators, and anti-neoplastics.

The effectiveness of the systemic medication is thus increased as a result of the provision of the localised heat gradient at the desired site on the human body.

In the present invention, all of the difficulties and drawbacks of the prior art systems, methods and procedures have been eliminated and a local, easily employed, convenient, consumer oriented treatment system for providing heat therapy for a wide variety of medical problems is achieved. In this invention, a treatment system is provided which comprises, at a minimum, a fully integrated, easily employed holding or support member which is uniquely constructed for cooperating with a heat delivery patch or exothermic pad for optimizing the application of heat directly to a precisely desired location.

In accordance with the present invention, an extremely novel, fully integrated treatment system is realized which possesses broad applicability for a wide range of medical conditions. In particular, medical conditions such as psoriasis, skin cancers, warts, leishmaniasis, mycobacteria, and granuloma annulare can be treated or improved by heat penetration into the skin, subcutaneous tissues, joints, muscles, blood streams, etc. As detailed herein, the application of heat has been found to slow, inhibit, or reverse metabolic processes, immunological processes, or biological conditions or processes which depend upon heat or are affected by heat. As a result, numerous medical conditions are effectively treated by employing the integrated treatment system of the present invention.

It has also been found that the use of heat produces a positive, synergistic effect on the targeted, controlled delivery of predetermined amounts of drugs, penetration enhancing agents, and/or cosmetics. In this regard, the topical use of drugs, penetration enhancing agents, and/or cosmetics for the treatment of skin conditions and/or subcutaneous symptoms including, but not limited to, pain, itch, and irritations are improved or effectively treated by the use of the present invention. Furthermore, the delivery of drugs, penetration enhancing agents and/or cosmetics through the skin for the purpose of achieving a non-oral and/or non-parenteral, systemic, transdermal delivery is effectively enhanced by the presence of a controlled heat gradient.

Exothermic pads or heat delivery patches have been previously developed and typically comprise a porous film or pad of woven or non-woven material incorporating chemicals which will react exothermically to generate heat in the presence of oxygen. Although any desired chemicals can be employed, exothermic pads or heat delivery patches typically contain moxa or a mixture of iron powder, activated charcoal, wood fibers, water and salt. Alternatively, a mixture of alkaline sulfides and iron carbide are employed with the chemicals stored in an inert, oxygen-free, chamber to prevent exposure to oxygen prior to use. In addition, the pores of the pad are of sufficient size to assure that the required air flow is achieved.

Prior to use, the patch/pad is typically sealed within a pouch with an inert gas, such as nitrogen. As long as the patch/pad remains in the sealed container until use, no chemical reaction takes place. However, once the pouch is open, the presence of the oxygen in the air causes the chemicals to react and the desired exothermic reaction is produced.

In the present invention, the exothermic pad or heat delivery patch is separately employed by having the user place the pad or patch on the desired site where heat treatment is being sought. Typically, the heat delivery patch or exothermic pad is separately secured to the desired site by adhesive means associated therewith. However, any desired securement method may be employed.

Once the patch/pad is secured in the precisely desired location, the support or holding member of the treatment system of the present invention is affixed to the desired location, peripherally surrounding and securely maintaining the heating pad or exothermic patch in the desired location, assisting in regulating and controlling the heat level and air transmission to the exothermic pad or heat delivery patch. In this way, the desired, controlled heat delivery or heat gradient is realized, enabling a broad range of medical conditions to be effectively treated.

As fully detailed below, in addition to providing controlled heat delivery for the direct treatment of certain medical conditions, the use of a heat gradient as provided by the fully integrated treatment system of the present invention is effective, improving and enhancing the penetration of systemic and topical medications such as corticosteroids, chemotherapeutic agents, anesthetics, antihistamines, anti-infectives, anti-bacterials, anti-parasitics, anti-viral agents, anti-oxidants, immunomodulators, and anti-neoplastics. Although a wide variety of chemical products come within the scope of these medications, the following list provides typical compositions that are effectively employed as a part of the treatment system of the present invention. However, this listing is provided for exemplary purposes only, and is not intended to limit the present invention thereto.

In this regard, corticosteroids may comprise one or more selected from the group consisting of hydrocortisone, triamcinolone, betamethasone, and any other steroids commonly used in topical applications to the skin; chemothera-peutic agents may comprise one or more selected from the group consisting of 5FU, Bleomycin, cytotoxic agents; anesthetics may comprise one or more selected from the group consisting of lidocaine, prilocaine, and pramoxine; antihistamines may comprise one or more selected from the group consisting of diphenhydramine and its salts; anti-infectives, such as anti-fungals, may comprise one or more selected from the group consisting of clotrimazole and ciclopirox; anti-bacterials may comprise one or more selected from the group consisting of gentamicin, tetracycline, erythromycin, and clindamycin; anti-parasitics may comprise one or more selected from the group consisting of metronidazole, permethrin, and crotamiton; anti-virals may comprise one or more selected from the group consisting of acyclovir; antioxidants may comprise one or more selected from the group consisting of ascorbic acid and tocopherol; immunomodulators may comprise one or more selected from the group consisting of imiquimod and beta glucan; and anti-neoplastics may comprise one or more selected from the group consisting of cytotoxic agents and immunomodulators.

Furthermore, it has also been discovered that the use of the heat gradient as provided by the present invention is effective in causing skin penetration enhancing agents to be more effectively employed. In this regard, as is fully detailed below, skin penetration enhancing agents which benefit from the use of the present invention comprise one or more selected from the group consisting of solvents, surfactants, ethers, esters, fatty acid glycerides, urea, oleates, liposomes, retinoids, and occlusive compounds.

As is evident from the foregoing, a wide variety of broadly diverse medical conditions are effectively and efficiently treated using the integrated treatment system of the present invention. As detailed above, this treatment system incorporates a heat delivery patch or exothermic pad in combination with a holding member which maintains the heat delivery patch or exothermic pad in a precisely desired location where the particular medical problem is manifested or where heat delivery or a heat gradient is desired. In addition, skin penetration enhancing agents and/or cosmetics may be integrated into the treatment system of this invention for further enhancement of its efficacy.

One area in which the use of heat has been widely documented is in the treatment of warts. In this regard, heat treatment for warts is fully disclosed in Dvoretzky, U.S. Patent 5,053,024. However, the use of heat in treating other skin disorders or medical conditions as well as the use of heat for assisting in the transmission and/or absorption of medicines, skin penetration enhancing agents, and/or cosmetics is unknown and represents an advance in this technological area.

Since the treatment of warts is one example of a medical problem that is effectively treated using the treatment system of this invention, the use of the present invention in connection with the treatment of warts is fully detailed herein. However, it is to be understood that the efficacious use of the present invention for treating warts is described herein for exemplary purposes only, and no limitation of the present invention to warts is intended. Furthermore, particular temperature levels and exposure times detailed herein are provided as examples of the present invention, and other temperatures or exposure times for other medical treatments can be attained with the present invention and are encompassed within the scope of the present invention.

By employing the treatment system of the present invention to treat warts, the wart virus is inactivated or slowed in its development, thereby preventing further invasion of the virus to adjacent tissue. As a result, the normal reparative process of the skin takes effect, moving in the infected portion to a higher and higher level into the spinous layer of the epidermis, towards the surface of the skin, until the wart is shed in the normal process with the stratum corneum.

By employing the treatment system of the present invention which comprises a holding member in combination with a heat delivery patch or exothermic pad, the temperature of the skin is elevated and maintained between about 39° to 44°C., for periods of time ranging between about 1 and 10 hours. In addition, by employing the present invention, the heat level to which the skin is exposed is maintained in a small, controlled range, which represents the optimum heat exposure for killing and inactivating the wart virus. As a result, optimum performance and treatment is realized.

If desired, keratolytic chemical agents may be incorporated with and/or into the exothermic pad to assist in reducing the thickness of the epidermis, particularly the stratum corneum. Inasmuch as the wart viruses are confined to the upper layer of the epidermis, the stratum granulosum and stratum corneum, any removal of the outermost horny layer technically eliminates a portion of the wart as well as the viral particles contained therein, destroying the viral particles by shedding. In addition, the thinning of the stratum corneum enables the heat to penetrate deeper within the wart lesion, thereby achieving greater efficacy from the application of the pad.

Although various keratolytic agents can be incorporated with and/or into the exothermic pad in order to reduce the upper layer of the epidermis, it has been found that salicylic acid or salicylic and lactic acid are preferable, since such chemicals may be incorporated with and/or into the exothermic pad to attain the desired beneficial results. By incorporating either salicylic acid, salicylic and lactic acid, or other similar chemical compositions, the exothermic pad may operate more effectively, since the thickness of the epidermis is reduced, thereby allowing the heat generated by the exothermic pad to penetrate the wart to a greater extent. In addition, immune response modifiers, such as interferon, imiquimod, DNCB, etc. may also be employed with equal efficacy.

In accordance with the present invention, the support member is specifically designed for being quickly and easily mountable to any location on an individual where a wart is normally found and remain in the precisely desired location in cooperating association with the heat delivery patch or exothermic pad. In addition, the support member cooperates with the heat delivery patch or exothermic pad to securely maintain the patch/pad in direct association with the wart. Furthermore, the support member of the present invention is also specifically constructed from material particularly designed for cooperating with a heat delivery patch or exothermic pad to synergistically interact therewith for assuring that the precisely required temperature levels are maintained and optimum oxygen flow is realized for the precisely desired period of time.

The support member forming the holding system of the present invention may be constructed in a variety of alternate configurations for providing the desired, quicky, easy, secure mounting thereof directly to any portion of the human anatomy where wart treatment is likely to be needed. In this regard, warts are most typically found on the extremities, thereby enabling the holding system of the present invention to comprise a construction which is quickly and easily mounted to an individual's hands, feet, toes, fingers, arms, forearms, legs, thighs, elbows, knees, nead, and torso.

In this regard, one preferred embodiment of the holding system of the present invention comprises a continuous, substantially cylindrically shaped tube member. By employing a cylindrically shaped member, the holding system of the present invention is able to be easily mounted onto the hands, feet, arms, legs, etc. of the user, peripherally surrounding the hands, feet, arms or legs, and securely maintaining and cooperating with the pre-mounted exothermic pad or heat delivery patch in the precisely desired location.

Although a cylindrically shaped holding system is preferred for most applications, alternate constructions can also be employed. Such alternate constructions include cones, truncated cones, closed-end cylinders, and elongated strips of various sizes incorporating fastening means, such as snaps, velcro, adhesives, etc. mounted at opposed ends for securing the holding system in peripheral surrounding engagement with any desired area of the human anatomy. In addition, if desired, tightening means, preferably in the form of an elongated strip member mounted on the outside surface of the cylindrical holding system, can be employed in order to assure secure, snug, mounted engagement of the treatment system in the precisely desired location.

One principal feature of the present invention is the material employed for forming the treatment system of this invention. In this regard, most woven or non-woven materials are capable of being employed for forming the holding member. However, it has been found that the preferred material comprises an elastomer with its entirety or formed therein, in order to provide flexibility in easily mounting and securing the holding system in place. In one preferred construction, the elastomer is integrally formed as a part of the material, such as a thermoplastic elastomer. In this regard, the preferred thermoplastic material comprises a foamed thermoplastic elastomer selected from the group consisting of polyurethane, polyolefins, polybutylenes, polyethylenes, polyesters, ethylene-propylene rubbers, polypronylenes, silicones, and vinyl based resins.

By employing a foamed thermoplastic elastomer, a precisely desired combination of closed cells and open cells is obtained, with the ratio of closed cells to open cells being controlled within a preferred range. In this way, the heat retention and/or insulation capabilities of the material are precisely maintained as well as the ability of the material to transmit oxygen from the surrounding, ambient air directly to the location of the heat delivery patch or exothermic pad.

In accordance with the present invention, the temperature delivered directly to the wart by the exothermic pad or heat delivery patch is precisely controlled in a narrow range, for a determined length of time, due to the insulation provided by the holding system. In addition, oxygen is circulated continuously through the holding system to provide proper operation of the exothermic pad/patch as well as provide air circulation to the surface of the skin being treated.

The invention accordingly comprises an article of manufacture possessing the features, properties, physical characteristics, qualities of the material being employed, and relation of elements which will be exemplified in the article hereinafter described and the scope of the invention will be indicated in the claims.

### THE DRAWINGS

For a fuller understanding of the nature and objects of the invention, reference should be had to the following detailed description taken in connection with the accompanying drawings, in which:
FIGURE 1 is a perspective view of one embodiment of the treatment system of the present invention with the holding member thereof mounted to a hand with an exothermic patch mounted to a finger of the hand;
FIGURE 2 is a perspective view of the holding member of FIGURE 1;
FIGURE 3 is a perspective view of an alternate construction of the holding member of the treatment system of the present invention;
FIGURE 4 is a perspective view of a further alternate embodiment of the holding member of the treatment system of the present invention; and
FIGURE 5 is a perspective view of a still further alternate embodiment of the holding member of the treatment system of the present invention.

### DETAILED DISCLOSURE

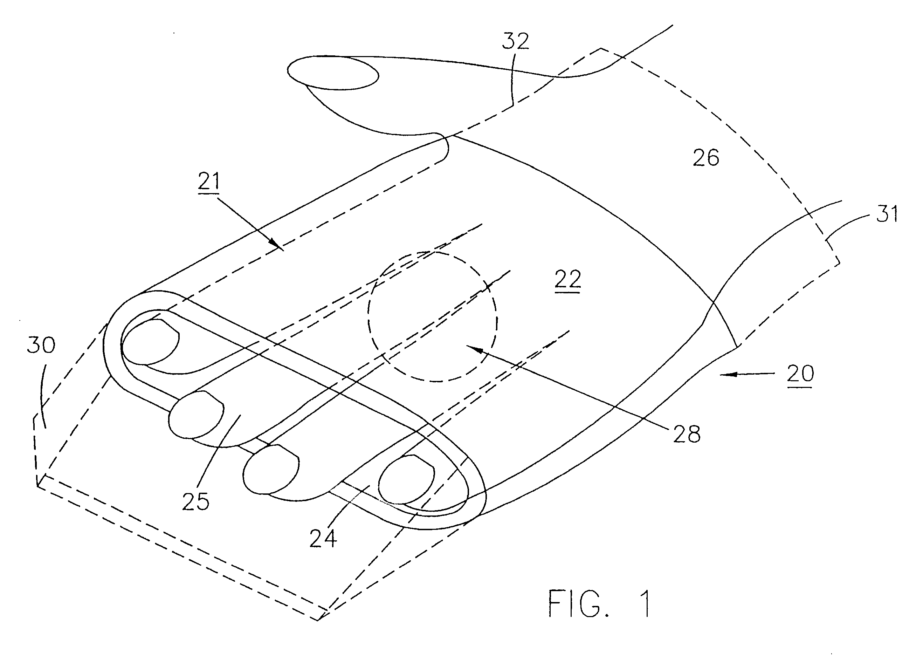

By referring to FIGURES 1-5, along with the following detailed disclosure, the construction and operation of the treatment system of the present invention can best be understood. In order to provide a full and complete teaching of the present invention, FIGURES 1-5 depict alternate preferred constructions for the treatment system of the present invention. However, it is to be understood that the embodiments are disclosed for exemplary purposes only, and are not intended as a limitation of the present invention. Consequently, alternate constructions and configurations for the system of the present invention can be made and are intended to be within this scope of the present invention.

In accordance with the present invention, treatment system 20 comprises a multi-component, fully integrated, easily employed construction for providing optimum delivery of heat therapy and/or a heat gradient to any desired location on the human anatomy for imparting medical benefits for a wide variety of various disorders and conditions. In this regard, in achieving an effective treatment system in accordance with the teaching of the present invention, treatment system 20 comprises holding member 21 and exothermic pad or heat delivery patch 28. In addition, if desired, any desired drugs, skin penetration enhancing agents and/or cosmetics may also be combined as part of treatment system 20 in order to attain desired enhanced medicinal benefits.

The present invention achieves an extremely novel, fully integrated treatment system having broad applicability for a wide range of medical conditions. As detailed herein, the application of heat has been found to slow, inhibit, or reverse metabolic processes, immunological processes, or biological conditions or processes, which depend upon heat or are affected by heat. As a result, broad applicability and extensive benefits are achieved by the present invention.

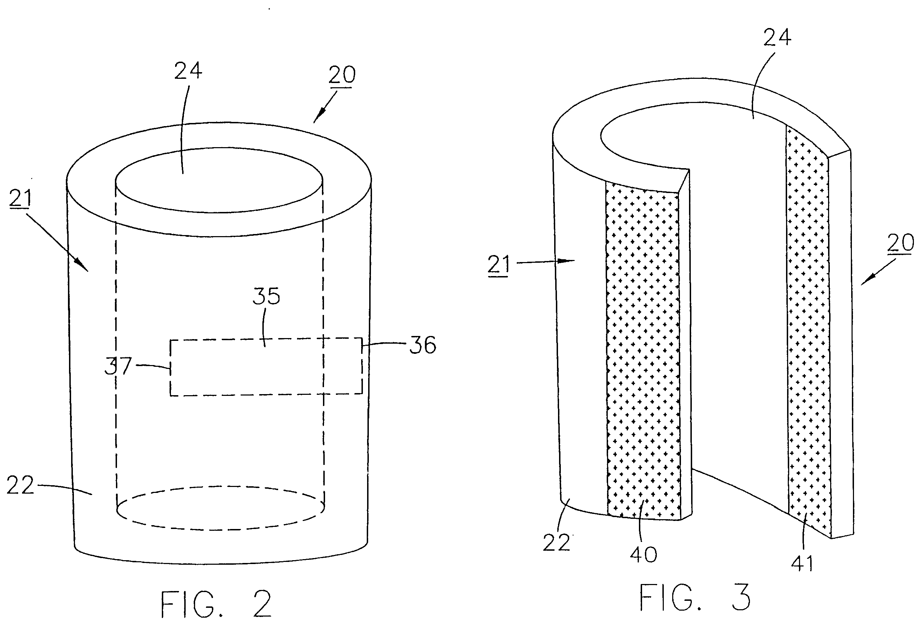

In FIGURES 2-3, alternate, exemplary embodiments of holding member 21 of treatment system 20 are depicted. As shown in FIGURES 1 and 2, one embodiment of treatment system 20 of the present invention is depicted as comprising a hollow, continuous, cylindrically shaped holding member 21 having an outer surface 22 and an inner surface 24. Although depicted as a hollow cylindrical member, holding member 21 may comprise any desired alternate size, shape, or configuration, and discussed herein.

In order to employ treatment system 20 of the present invention in a manner which will provide controlled heating of any desired site where treatment is being sought, reference should be made to FIGURE 1. In this Figure, the existence of a wart on finger 25 of hand 26 is depicted for exemplary purposes, with exothermic pad or heat delivery patch 28 mounted to finger 25 in overlying engagement with the wart formed thereon. In addition, treatment system 20 of the present invention is placed about the fingers of hand 26, in overlying, secure, co-operative engagement with exothermic pad 28.

By securely mounting holding system 20 in overlying, cooperative engagement with heat delivery patch or exothermic pad 28, holding system 20 provides the desired co-operative association with pad/patch 28 to assure controlled heat delivery and oxygen circulation directly to the desired location. In this way, protection of the desired site is provided, along with control of the temperature to which the skin surface is exposed. As a result, the precisely desired temperature is delivered to the wart, within a desired, narrow range and for the precise length of time needed for killing the wart virus.

In addition to its efficacy for treating warts, treatment system 20 of the present invention is also capable of treating other medical conditions such as psoriasis, skin cancers, leishmaniasis, mycobacteria, and granuloma annulare. Each of these conditions can be treated or improved by heat penetration into the skin, subcutaneous tissues, joints, muscles, blood streams, etc. The application of heat has been found to slow, inhibit, or reverse metabolic processes, immunological processes, or biological conditions or processes which depend upon heat or are affected by heat. As a result, numerous medical conditions are effectively treated by employing the integrated treatment system of the present invention.

It has also been found that the use of heat produces a positive, synergistic effect on the targeted, controlled delivery of predetermined amounts of drugs, skin penetration enhancing agents, and/or cosmetics. In this regard, the topical use of drugs, penetration enhancing agents, and/or cosmetics for the treatment of skin conditions and/or subcutaneous symptoms including, but not limited to, pain, itch, and irritations, are improved or effectively treated by the use of the present invention. Furthermore, the delivery of drugs, penetration enhancing agents and/or cosmetics through the skin for the purpose of achieving a non-oral and/or non-parenteral, systemic, transdermal delivery is effectively enhanced by the presence of a controlled heat gradient provided by the present invention.

It has been found that the use of a heat gradient, as provided by the fully integrated treatment system of the present invention, is effective, in improving and enhancing the penetration of systemic and topical medications such as corticosteroids, chemotherapeutic agents, anesthetics, antihistamines, anti-infectives, anti-bacterials, anti-parasitics, anti-viral agents, anti-oxidants, immunomodulators, keratolytics, and anti-neoplastics. Although a wide variety of chemical products come within the scope of these medications, the following list provides typical compositions that are effectively employed as a part of the treatment system of the present invention. However, this listing is provided for exemplary purposes only, and is not intended to limit the present invention thereto.

In this regard, corticosteroids may comprise one or more selected from the group consisting of hydrocortisone, triamcinolone, and betamethasone and any other steroid commonly used in topical applications to the skin; chemothera-peutic agents may comprise one or more selected from the group consisting of 5FU, Bleomycin, cytotoxic agents; anesthetics may comprise one or more selected from the group consisting of lidocaine, prilocaine and pramoxine; antihistamines may comprise one or more selected from the group consisting of diphenhydramine and its salts; anti-infectives, such as anti-fungals, may comprise one or more selected from the group consisting of clotrimazole and ciclopirox; anti-bacterials may comprise one or more selected from the group consisting of gentamicin, tetracycline, erythromycin, and clindamycin; anti-parasitics may comprise one or more selected from the group consisting of metronidazole, permethrin, and crotamiton; anti-virals may comprise one or more selected from the group consisting of acyclovir; anti-oxidants may comprise one or more selected from the group consisting of ascorbic acid and tocopherol: immunomodulators may comprise one or more selected from the group consisting of imiquimod and beta glucan; keratolytics may comprise one or more selected from the group consisting of salicylic acid; and anti-neoplastics may comprise one or more selected from the group consisting of cytotoxic agents and immunomodulators.

Furthermore, it has also been discovered that the use of the heat gradient as provided by the present invention is effective in causing skin penetration enhancing agents to be more effectively employed. In this regard, skin penetration enhancing agents which benefit from the use of the present invention comprise one or more selected from the group consisting of solvents, surfactants, ethers, esters, fatty acid glycerides, urea, oleates, liposomes, retinoids, and occlusive compounds.

Although a wide variety of skin penetrating enhancing agents can be effectively employed as an integral part of the treatment system 20 of the present invention, the following listing is provided as examples of the various types of agents that may be effectively employed as part of the present invention. In this regard, solvents may comprise one or more selected from the group consisting of dimethyl sulfoxide, dimethyl acetamide, dimethyl formamide, diethyl-m-toluamide, and n-methyl pyrrolidone; alcohols may comprise one or more selected from the group consisting of ethanol and isopropanol; glycols may comprise one or more selected from the group consisting of propylene glycol, polyethylene glycols, and glycerin; ketones may comprise one or more selected from the group consisting of acetone; surfactants, including anionic surfactants, may comprise one or more selected from the group consisting of sodium lauryl sulfate; surfactants, including non-ionic surfactants, may comprise one or more selected from the group consisting of laureth-4, polysorbates, poloxamers; surfactants, including cationic surfactants, may comprise one or more selected from the group consisting of stearylkonium chloride and benzalkonium chloride; ethers, including organic ethers, may comprise one or more selected from the group consisting of diethylene glycol monoethyl ether, polyoxypropylene 15 stearly ether, and dimethyl ether of isosorbide; esters , including organic esters, may comprise one or more selected from the group consisting of isopropyl myristate, isopropyl palmitate, ethyl acetate, and C 12-15 alkyl benzoate; fatty acid glycerides may comprise one or more selected from the group consisting of glyceryl laurate and glyceryl stearate; urea; oleates may comprise one or more selected from the group consisting of oleyl alcohol, oleic acid, and sorbitan sesquioleate; liposomes; imidazoles may comprise one or more selected from the group consisting of sulconazole nitrate; dioxolones; caprolactames may comprise one or more selected from the group consisting of 1-dodecylazacycloheptan-2-one; hydroxyacids may comprise one or more selected from the group consisting of glycolic acid, lactic acid, and salicylic acid; retinoids may comprise one or more selected from the group consisting of retinol and all-trans-retinoic acid; and occlusive compounds may comprise one or more selected from the group consisting of petrolatum, occlusion with plastic film, occlusion with film forming polymers such as collodion, ethyl cellulose, and cellulose acetate.

As is evident from the foregoing discussion, the present invention provides a treatment system for a wide variety of medical problems and conditions. As a result, by employing the treatment system of this invention, controlled delivery of heat therapy and/or a heat gradient is provided to any desired location on the human anatomy for imparting medical benefits for a wide variety of various disorders and conditions. In this way, both medicinal benefits and drug delivery are substantially enhanced.

As detailed above, the treatment system of the present invention comprises holding member 21 and exothermic pad or heat delivery patch 28, along with any desired drug, skin penetration enhancing agent and/or cosmetic which may be desired for combination therewith, as a part of treatment system 20. In addition, holding member 21 may be formed in virtually any desired size or configuration. However, for exemplary purposes only, FIGURES 1-5 depict preferred alternate configurations for holding member 21 of treatment system 20. As previously discussed, holding member 21 is depicted in FIGURES 1 and 2 as a hollow, continuous, cylindrically shaped member which is easily mountable to virtually any desired location on an extremity of an individual. By constructing holding member 21 from various flexible materials, an easy mounted construction is achieved, providing the precisely desired heat therapy and/or heat gradient in virtually any desired location. In this regard, it has been found that woven or nonwoven materials can be employed for forming holding member 21. In addition, holding member 21 may be constructed with elastomeric materials in order to provide further enhanced flexibility and mounting and securing ease.

In one preferred construction, holding member 21 is formed from thermoplastic elastomeric materials selected from the group consisting of polyurethanes, polyolefins, polybutylenes, polyethylenes, polyesters, ethylene-propylene rubbers, polypropylenes, silicones, and vinyl-based resins. Furthermore, by constructing holding member 21 from a foamed thermoplastic elastomer, any desired combination of closed cells and open cells is obtainable, with any desired ratio being provided. In this way, heat retention and/or insulation capabilities are achieved as an integral, inherent physical characteristic of holding member 21. In addition, by employing this construction, holding member 21 is able to transmit oxygen from the surrounding, ambient air directly to the location where the heat delivery patch or exothermic pad 28 is mounted.

As discussed above, FIGURE 1 depicts holding member 21 mounted about the hand of a user, peripherally surrounding all four fingers, with exothermic pad or heat delivery patch 28 mounted to finger 25 in aligned, overlying relationship with the area to be treated. In addition, as shown in phantom, holding member 21 may comprise forwardly projecting portion 30 which extends forward of the fingertips of the user in order to allow the fingertips to be wrapped and enclosed, if desired. In addition, rearwardly extending portion 31 is also depicted in FIGURE 1 for showing an elongated construction incorporating a thumb finger hole 32 formed therein for ease of application and mounting to the hand of an individual.

In FIGURE 2, cylindrically shaped holding member 21 is depicted incorporating an optional securing strap 35 mounted to outer surface 22 thereof. Although securing strap 35 may incorporate a wide variety of alternate constructions, in the preferred embodiment, strap 35 comprises a continuous, elongated member having opposed ends 36 and 37, with end 36 integrally bonded to outer surface 22 of holding member 21. In addition, opposed end 37 of strap 35 is constructed for being securable to surface 22 in a wide variety of alternate locations.

In order to provide the desired securability for strap 35 with surface 22 of holding member 21, fastening means are preferably formed adjacent end 37 of strap 35, positioned for cooperative interengagement with surface 22 of holding member 21. In this regard, any desired fastening means may be employed, such as adhesives, hook/loop fasteners, and the like.

Preferably, if employed strap 35 provides the desired securement of holding member 21 in the precisely desired location. In this regard, holding member 21 is mounted about the desired area to which the heat therapy or heat gradient is to be delivered and end 37 of strap 35 is removed from its initial engagement with surface 22 and moved into an alternate engaged location engage with surface 22 which provides the desired secure positioning of holding member 21 in the precisely desired location.

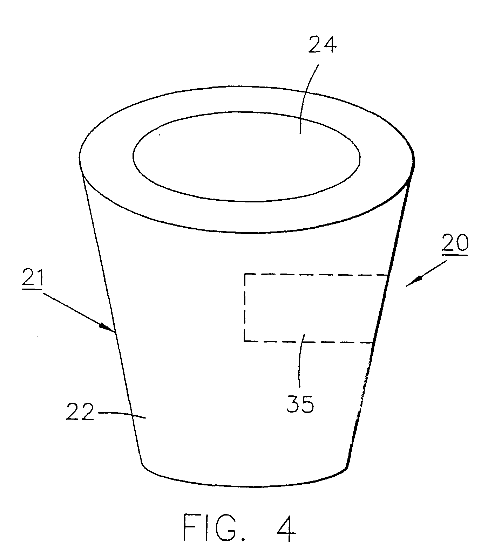

In FIGURE 3, an alternate embodiment of holding member 21 of treatment system 20 of the present invention is depicted. In this embodiment, an elongated, substantially continuous sheet of material is employed which is easily wrapped about any desired location for peripherally surrounding an exothermic pad or heat delivery patch 28 mounted to the desired site. In order to assure ease of securement of holding member 21 in the precisely desired location, fastening means 40 are formed on outer surface 22 of holding member 21, with cooperating fastening means 41 formed on inner surface 24 of holding member 21. Although fastening means 40 and 41 are depicted as hook and loop fasteners, any alternate fastening means may be employed with equal efficacy.

In this embodiment, holding member 21 may be constructed in any desired size and shape for enabling holding member 21 to be mounted to virtually any locations on the human anatomy. In this way, treatment system 20 of the present invention can be effectively employed wherever needed for providing the desired heat therapy or heat gradient, as well as the enhanced delivery of any medicines, skin penetration enhancing agents, and/or cosmetics.

In FIGURE 4, a further alternate embodiment of holding member 21 of treatment system 20 of the present invention is depicted. In this embodiment, holding member 21 is constructed in a substantially continuous, hollow, truncated conical shaped. By employing this configuration, ease of mounting and securement of holding member 21 to particular locations is enhanced. Furthermore, if desired, this embodiment of holding member 21 may be constructed with securing strap 35 mounted to outer surface 22 thereof, as detailed above. In addition, this embodiment of holding member 21 may also be constructed with an elongated slit formed in the outer surface thereof, as depicted in FIGURE 3, while also incorporating fastening means 40 and 41 formed on the surfaces thereof for enabling secure wrapped interengagement of holding member 21 to any desired location.

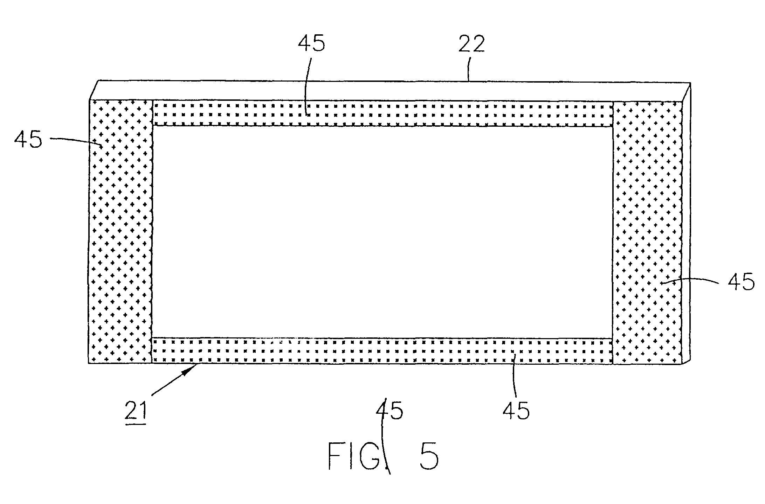

In FIGURE 5, an alternate embodiment of holding member 21 of treatment system 20 of he present invention is depicted. In this embodiment, an elongated, substantially continuous sheet of material is employed which is quickly and easily affixed to any desired surface location on an individual for securely maintaining an exothermic paid or heat delivery patch 28 on the desired site. In order to assure ease of securement of holding member 21 in the precisely desired location, adhesive means 45 are formed on inner surface 24 of holding member 21. along at least two opposed edges. If desired, adhesive means 45 may be formed about the entire peripheral edge of surface 24 to assure secure affixation of holding member 21 to any surface of the body, without requiring peripheral surrounding of the body part. Although fastening means 40 and 41 are depicted as hook and loop fasteners, any alternate fastening means may be employed with equal efficacy.

In this embodiment, holding member 21 may be constructed in any desired size and shape for enabling holding member 21 to be mounted to virtually any location on the human anatomy. In this way, treatment system 20 of the present invention can be effectively employed whenever needed for providing the desired heat therapy or heat gradient, as well as the enhanced delivery of any medicines, skin penetration enhancing agents, and/or cosmetics.

As is evident from the foregoing detailed discussion, holding member 21 of treatment system 20 of the present invention may be configured in a wide variety of alternate constructions without departing from the scope of the present invention. However, regardless of the configuration employed, holding member 21 peripherally surrounds a pre-positioned exothermic pad or heat delivery patch 28 which has been mounted to the particular site where medicinal benefits are desired. In addition, by also placing drugs, skin penetration enhancing agents and/or cosmetics on the site being treated, further enhanced medicinal benefits may be achieved with treatment system 20 of this invention.

It will must be seen that the object set forth above, among those made apparent from the preceding description, are efficiently obtained and, since certain changes may be made in the above article without departing from the scope of the invention as defined by the appended claims, it is intended that all matter contained in the above description or shown in the accompanying drawings shall be interpreted as illustrative and not in a limiting sense.

It is also to be understood that the following claims are intended to cover all of the generic and specific features of the invention herein described, and all statements of the scope of the invention which, as a matter of language, might be said to fall therebetween.

Having described our invention, what we what we claim as new and desired to secure by Letters Patent is:

## Claims

1. A treatment device comprising a holding and supporting member (21) constructed for being retained on a desired site of a human body and a heat generating member (28) positioned in cooperating relationship with the holding and supporting member (21) for enabling the application of heat directly to the desired site, **characterised by** the device further comprising a systemic medication that exhibits improved or enhanced penetration from the application of a heat gradient thereto and is selected from the group consisting of corticosteroids, chemotherapeutic agents, antihistamines, anti-parasitics, anti-oxidants, immunomodulators, and anti-neoplastics.

2. A treatment device as claimed in Claim 1, in which the systemic medication is a corticosteroid selected from the group consisting of hydrocortisone, triamcinolone, betamethasone, and any other steroids commonly used in topical applications to the skin.

3. A treatment device as claimed in Claim 1, in which the systemic medication is a chemotherapeutic agent selected from the group consisting of 5FU, Bleomycin, and acytotoxic agents.

4. A treatment device as claimed in Claim 1, in which the systemic medication is an antihistamine selected from the group consisting of diphenhydramine and its salts.

5. A treatment device as claimed in Claim 1, wherein the systemic medication is an anti-parasitic selected from the group consisting of metronidazole, permethrin, and crotamiton.

6. A treatment device as claimed in Claim 1, wherein the systemic medication is an antioxidant selected from the group consisting of ascorbic acid and tocopherol.

7. A treatment device as claimed in Claim 1, wherein the systemic medication is an immunomodulator selected from the group consisting of imiquimod and beta glucan.

8. A treatment device as claimed in Claim 1, wherein the systemic medication is an anti-neoplastic selected from the group consisting of cytotoxic agents and immunomodulators.

9. A treatment device as claimed in any of the preceding claims, which further comprises a skin enhancing agent incorporated therewith for benefiting from the heat gradient.

10. A treatment device as claimed in Claim 9, wherein said skin penetration enhancing agent is selected from the group consisting of solvents, surfactants, ethers, esters, fatty acid glycerides, urea, oleates, liposomes, retinoids, and occlusive compounds.

11. A treatment device as claimed in any of the preceding claims, wherein said holding member comprises a hollow, continuous, generally cylindrically shaped member.

12. A treatment device as claimed in Claim 11, wherein said holding and supporting member (21) has a diameter configured for secure, surrounding engagement with a particular part of the human anatomy.

13. A treatment device as claimed in Claim 12, wherein the holding and supporting member (21) is constructed for mounting securement to a body part selected from the group consisting of fingers, arms, elbows, toes, feet, legs, wrists, ankles, and the upper torso.

14. A treatment device as claimed in Claim 13, wherein the hollow cylindrical shape is either a regular cylinder or a truncated cone.

15. A treatment device as claimed in Claim 14, **characterised by** the provision of an elongated strap (35) for enabling tightening of the holding and supporting member (21) in the desired location.

16. A treatment device as claimed in any of the preceding claims, **characterised in that** the holding and supporting member (21) comprises an elongated, substantially planar construction incorporating fastening means for securing said member (21) in any desired location.

17. A treatment device as claimed in any one of the preceding claims, **characterised in that** the holding and supporting member (21) is formed from thermoplastic elastomeric materials.

18. A treatment device as claimed in Claim 17, **characterised in that** the thermoplastic elastomeric material is selected from the group consisting of polyurethanes, polyolefins, polybutylenes, polyethylenes, polyesters, ethylene-propylene rubbers, polypropylenes, silicones, and vinyl-based resins.

## Patentansprüche

1. Behandlungsvorrichtung, umfassend ein Halte- und Stützelement (21), das so konstruiert ist, dass es auf einer gewünschten Stelle eines menschlichen Körpers gehalten werden kann, und ein Wärmeerzeugungselement (28), das so positioniert ist, dass es mit dem Halte- und Stützelement (21) zusammenarbeitet, um die Aufbringung von Wärme direkt auf die gewünschte Stelle zu ermöglichen, **dadurch gekennzeichnet, dass** die Vorrichtung ferner ein systemisches Medikament umfasst, das verbesserte oder verstärkte Penetration nach dem Aufbringen eines Wärmegradienten darauf aufweist und aus der aus Kortikosteroiden, Chemotherapeutika, Antihistaminika, Antiparasitika, Antioxidantien, Immunmodulatoren und Antineoplastika bestehenden Gruppe ausgewählt ist.

2. Behandlungsvorrichtung nach Anspruch 1, bei der das systemische Medikament ein Kortikosteroid ist, das aus der aus Hydrokortison, Triamcinolon, Betamethason und jedem anderen Steroid, das üblicherweise bei topischen Hautapplikationen zur Anwendung kommt, bestehenden Gruppe ausgewählt ist.

3. Behandlungsvorrichtung nach Anspruch 1, bei der das systemische Medikament ein Chemotherapeutikum ist, das aus der aus 5FU, Bleomycin und acytotoxischen Mitteln bestehenden Gruppe ausgewählt ist.

4. Behandlungsvorrichtung nach Anspruch 1, bei der das systemische Medikament ein Antihistaminikum ist, das aus der ausd Diphenhydramin und seinen Salzen bestehenden Gruppe ausgewählt ist.

5. Behandlungsvorrichtung nach Anspruch 1, bei der das systemische Medikament ein Antiparasitikum ist, das aus der aus Metronidazol, Permethrin und Crotamiton bestehenden Gruppe ausgewählt ist.

6. Behandlungsvorrichtung nach Anspruch 1, bei der das systemische Medikament ein Antioxidans ist, das aus der aus Ascorbinsäure und Tocopherol bestehenden Gruppe ausgewählt ist.

7. Behandlungsvorrichtung nach Anspruch 1, bei der das systemische Medikament ein Immunmodulator ist, der aus der aus Imiquimod und Beta-Glucan bestehenden Gruppe ausgewählt ist.

8. Behandlungsvorrichtung nach Anspruch 1, bei der das systemische Medikament ein antineoplastisches Mittel ist, das aus der aus Zytotoxika und Immunmodulatoren bestehenden Gruppe ausgewählt ist.

9. Behandlungsvorrichtung nach einem der vorhergehenden Ansprüche, die ferner ein darin eingearbeitetes Hautverbesserungsmittel umfasst, um vom Wärmegradienten zu profitieren.

10. Behandlungsvorrichtung nach Anspruch 9, worin das Mittel zur Verbesserung der Hautpenetration aus der aus Lösungsmitteln, Tensiden, Ethern, Estern, Fettsäureglyceriden, Harnstoff, Oleaten, Liposomen, Retinoiden und okklusiven Verbindungen bestehenden Gruppe ausgewählt ist.

11. Behandlungsvorrichtung nach einem der vorhergehenden Ansprüche, worin das Halteelement ein hohles, kontinuierliches, allgemein zylinderförmiges Element umfasst.

12. Behandlungsvorrichtung nach Anspruch 11, worin das Halte- und Stützelement (21) einen Durchmesser aufweist, der für den sicheren umgebenden Eingriff mit einem bestimmten Teil der menschlichen Anatomie konfiguriert ist.

13. Behandlungsvorrichtung nach Anspruch 12, worin das Halte- und Stützelement (21) so konstruiert ist, dass es sicher am einem aus Fingern, Armen, Ellbogen, Zehen, Füßen, Beinen, Handgelenken, Fußgelenken und Oberkörper ausgewählten Körperteil befestigt werden kann.

14. Behandlungsvorrichtung nach Anspruch 13, worin die hohle Zylinderform entweder einen regelmäßigen Zylinder oder einen Kegelstumpf darstellt.

15. Behandlungsvorrichtung nach Anspruch 14, **gekennzeichnet durch** die Bereitstellung eines länglichen Gurts (35) zum Anziehen des Halte- und Stützelements (21) am gewünschten Ort.

16. Behandlungsvorrichtung nach einem der vorhergehenden Ansprüche, **dadurch gekennzeichnet, dass** das Halte- und Stützelement (21) eine längliche, im Wesentlichen ebene Konstruktion mit Befestigungsmitteln zum Befestigen des Elements (21) an jedem gewünschten Ort umfasst.

17. Behandlungsvorrichtung nach einem der vorhergehenden Ansprüche, **dadurch gekennzeichnet, dass** das Halte- und Stützelement (21) aus thermoplastischen elastomeren Materialien geformt ist.

18. Behandlungsvorrichtung nach Anspruch 17, **dadurch gekennzeichnet, dass** das thermoplastische elastomere Material aus der aus Polyurethanen, Polyolefinen, Polybutylenen, Polyethylenen, Polyestern, Ethylen-Propylen-Kautschuken, Polypropylenen, Silikonen und Harzen auf Vinylbasis bestehenden Gruppe ausgewählt ist.

## Revendications

1. Dispositif de thérapie comprenant un élément de maintien et de support (21) prévu pour être retenu sur un site souhaité d'un corps humain et un élément produisant de la chaleur (28) positionné en relation de coopération avec l'élément de maintien et de support (21) pour permettre l'application de chaleur directement sur le site souhaité, **caractérisé par le fait que** le dispositif comprend en outre un médicament systémique qui présente une pénétration améliorée ou accrue par l'application d'un gradient de température sur celui-ci et qui est sélectionné parmi le groupe constitué des corticostéroïdes, des agents chimiothérapeutiques, des antihistamines, des antiparasitiques, des antioxydants, des immunomodulateurs et des antinéoplastiques. '

2. Dispositif de thérapie selon la revendication 1, dans lequel le médicament systémique est un corticostéroïde choisi parmi le groupe constitué de l'hydrocortisone, de la triamcinolone, de la bétaméthasone, et de tout autre stéroïde utilisé couramment dans les applications topiques sur la peau.

3. Dispositif de thérapie selon la revendication 1, dans lequel le médicament systémique est un agent chimiothérapeutique choisi parmi le groupe constitué de 5FU, bléomycine et d'agents acytotoxiques.

4. Dispositif de thérapie selon la revendication 1, dans lequel le médicament systémique est une antihistamine choisie parmi le groupe constitué de la diphénhydramine et de ses sels.

5. Dispositif de thérapie selon la revendication 1, dans lequel le médicament systémique est un antiparasitique choisi parmi le groupe constitué du métronidazole, de la perméthrine et du crotamiton.

6. Dispositif de thérapie selon la revendication 1, dans lequel le médicament systémique est un antioxydant choisi parmi le groupe constitué de l'acide ascorbique et du tocophérol.

7. Dispositif de thérapie selon la revendication 1, dans lequel le médicament systémique est un immunomodulateur choisi parmi le groupe constitué de l'imiquimod et du bétaglucane.

8. Dispositif de thérapie selon la revendication 1, dans lequel le médicament systémique est un antinéoplastique choisi parmi le groupe constitué d'agents cytotoxiques et d'immunomodulateurs.

9. Dispositif de thérapie selon l'une quelconque des revendications précédentes, comprenant en outre un agent d'amélioration de la pénétration à travers la peau incorporé dans celui-ci de manière à bénéficier du gradient de température.

10. Dispositif de thérapie selon la revendication 9, dans lequel ledit agent d'amélioration de la pénétration à travers la peau est choisi parmi le groupe constitué de solvants, tensioactifs, éthers, esters, glycérides d'acides gras, urée, oléates, liposomes, rétinoïdes, et composés occlusifs.

11. Dispositif de thérapie selon l'une quelconque des revendications précédentes, dans lequel ledit élément de maintien comprend un élément creux continu, de forme généralement cylindrique.

12. Dispositif de thérapie selon la revendication 11, dans lequel ledit élément de maintien et de support (21) a un diamètre configuré de manière à fournir un engagement sûr d'enveloppement avec une partie particulière de l'anatomie humaine.

13. Dispositif de thérapie selon la revendication 12, dans lequel l'élément de maintien et de support (21) est prévu pour être fixé par montage sur une partie corporelle appartenant au groupe constitué des doigts, des bras, des coudes, des orteils, des pieds, des jambes, des poignets, des chevilles et de la partie supérieure du torse.

14. Dispositif de thérapie selon la revendication 13, dans lequel la forme cylindrique creuse est un cylindre régulier ou un cône tronqué.

15. Dispositif de thérapie selon la revendication 14, **caractérisé par** la fourniture d'une bande allongée (35) destinée à permettre de serrer l'élément de maintien et de support (21) dans l'emplacement souhaité.

16. Dispositif de thérapie selon l'une quelconque des revendications précédentes, **caractérisé en ce que** l'élément de maintien et de support (21) comprend une construction allongée substantiellement plane incorporant des moyens d'attache pour fixer ledit élément (21) dans un emplacement souhaité quelconque.

17. Dispositif de thérapie selon l'une quelconque des revendications précédentes, **caractérisé en ce que** l'élément de maintien et de support (21) est formé à partir de matériaux thermoplastiques élastomères.

18. Dispositif de thérapie selon la revendication 17, **caractérisé en ce que** le matériau thermoplastique élastomère est choisi parmi le groupe constitué des polyuréthanes, des polyoléfines, des polybutylènes, des polyéthylènes, des polyesters, des caoutchoucs éthylène-propylène, des polypropylènes, des silicones et des résines à base de vinyle.
